# EUROPEAN PATENT APPLICATION

(11) **EP 2 348 318 A1**
(43) Date of publication of application: **27.07.2011**
(21) Application number: 10382011.4
(22) Date of filing: 21.01.2010
(51) Int. Cl.: G01N 33/88

(54) **Diagnostic method for endometrial receptivity**

(71) Applicant: EQUIPO IVI INVESTIGACIÓN, S.L., 46015 Valencia (ES)
(72) Inventor: Simon Vallès, Carlos, E-46015, Valencia (ES); Pellicer Martinez, Antonio, E-46015, Valencia (ES); Berlanga Atienza, Óscar, E-46015, Valencia (ES)
(74) Representative: ABG Patentes, S.L.

(57) **Abstract**

The invention relates to a non-invasive diagnostic method of endometrial receptivity which includes collecting and comparing lipid biomarker patterns to reference lipidomic markers, particularly prostaglandins PGE2 and/or PGF2α. The method is especially applicable for determining status fertility of a mammalian female, preferably, a human female.

## Description

### FIELD OF THE INVENTION

The invention relates to a non-invasive diagnostic method of endometrial receptivity. The method is especially applicable for determining status fertility of a mammalian female, preferably, a human female. The method includes collecting and comparing lipid biomarker patterns to reference lipidomic markers. This invention further relates to a kit for performing said diagnostic method.

### BACKGROUND OF THE INVENTION

An estimated 12% of couples attempting to conceive suffer from infertility, and because of the increasing age of pregnancy this figure is on the rise. *In vitro* fertilisation (IVF) has been used ever more to assist infertile couples in becoming pregnant. Since the first successful IVF treatment in 1978 that led to the birth of Louise Brown, many technical and medical advances have contributed to improve efficiencies; nowadays, around 70% of women undergoing IVF treatment have successful outcomes. Still some 50% of patients do not become pregnant, or have early pregnancy loses, which calls for further efforts among the scientific community to continue research into the field.

IVF generally is a multistep process that involves recovering mature eggs from the female patient or donor; incubation of eggs in artificial culture media; collection of sperm from the patient or donor and subsequent preparation; fertilization of the egg by the sperm; monitoring and selection of good-quality embryos; and transfer of these to the uterine cavity. The embryo must implant onto the receptive endometrium so that pregnancy can progress. It is well established that this particular step is responsible for a significant percentage of the failures, and, therefore, it is clinically relevant to look at possible causes and solutions of the problem.

Implantation of the transferred embryo involves a synchronized crosstalk between a receptive endometrium and a functional blastocyst [Wang, H., and Dey, SK. 2006. Roadmap to embryo implantation: clues from mouse models. Nature Review Genetics. 7:185-199]. This phenomenon can only take place during the window of implantation, a self-limited period of endometrial receptivity spanning between days 19 and 23 of the menstrual cycle (women). In normal menstrual cycles this is achieved through the local effects of ovarian estrogens and progesterone, which induce a series of cellular and molecular events in the endometrium leading to appropriate endometrial receptivity. The latter is assessed using the Noyes criteria for endometrial dating, which was proposed over 50 years ago [Noyes, RW., Hertig, AJ., and Rock, J. 1950. Dating the endometrial biopsy. Fertil Steril, 1:3-25] but is still the preferred method used today to measure endometrial receptivity. Today, in the light of 15 years of experience with donor-egg IVF, it is accepted that histological evaluation adds little clinically significant information, and should be replaced by functional assessment of endometrial receptivity.

The era of molecular and cellular biology, and the development of new analytical techniques, has aided the quest for more consistent predictors of a receptive endometrium. Upon induction with steroid hormones, a large number of structural and molecular mediators have been identified to date that make potential markers of endometrial receptivity, including the presence of pinopodes on the surface of endometrial cells, adhesion molecules such as integrins, cytokines, growth factors, lipids and others [Nikas, G., and Aghajanova. 2002. Endometrial pinopodes: some more understanding on human implantation? Reprod Biomed Online 4 Suppl. 3:18-23; Lessey, BA. 2003. Two pathways of progesterone action in the human endometrium: implications for implantation and contraception. Steroids. 68:809-815; Robb L, Dimitriadis E, Li R, Salamonsen LA., 2002. Leukemia inhibitory factor and interleukin-11: cytokines with key roles in implantation. J Reprod Immunol. 57: 129-141].

Furthermore, genetic and proteomic analysis have added new exciting tools for the assessment of endometrial receptivity, and endometrial biopsy samples can be used to identify molecules associated with uterine receptivity to obtain a better insight into human implantation. These approaches, however, have not yet rendered results applicable to the everyday-IVF clinical practice, since no single specific factor has been identified to be crucial for implantation in humans [Strowitzki, T., Germeyer, A., Popovici, R., et al. 2006. The human endometrium as a fertility-determining factor. Human Reproduction Update. 12:617-630]. Furthermore, a major setback in the application of biopsy-dependent techniques lies on their invasive nature, and also in the necessity of appropriately timed endometrial biopsies that disrupt the necessary endpoint of implantation [van der Gaast, MH., Beier-Hellwig, K., Fauser, BC., et al. 2003. Endometrial secretion aspiration prior to embryo transfer does not reduce implantation rates. Reprod Biomed Online. 7:105-109].

The analysis of endometrial secretions is a new, non-disruptive possibility for the investigation of endometrial receptivity. Importantly, the aspiration of endometrial fluid does not affect pregnancy rates [van der Gaast et al., 2003, cited *supra*]*.* This approach provides reliable read-outs of individual proteins correlating with day of cycle and has proven efficient in protein arrays using a luminex system [Boomsma, CM., Kavelaars, A., Eijkemans, MJC., et al. 2009. Cytokine profiling in endometrial secretions: a non invasive window on endometrial receptivity. Reprod Biomed Online. 18:85-94], thus opening the field for a non-invasive application.

Although some biochemical markers have been disclosed by assessing endometrial receptivity [Giudice, L.C. 1999. Hum Reprod 14 Suppl 2:3-16; Achache, H et al.; Human Reproduction Update, 2006; 12:731-46, Thomas, K et al.; Fertil. Steril. 2003; 80:502-507; WO03062832], there exists the need of identifying alternative efficient biomarkers, differentially produced during the implantation window, which can be used in clinic.

Metabolomics is a discipline dedicated to the systematic study of small molecules (i.e., metabolites) in cells, tissues, and biofluids. Metabolites are the end products of cellular regulatory processes, and their levels can be regarded as the amplified response of biological systems to genetic or environmental changes. Clinicians have relied for decades on a small part of the information contained in the metabolome, for example measuring glucose to monitor diabetes and measuring cholesterol for cardiovascular health. New sophisticated metabolomic analytical platforms and informatic tools have already been developed that afford extended and sensitive measurement of the metabolome.

Lipids are known to play an important role as structural components (e.g., cell membranes), energy storage components, and as signalling molecules. Lipids are broadly defined as hydrophobic or amphipathic small molecules that may originate entirely or in part by carbanion based condensation of thioesters, and/or by carbocation based condensation of isoprene units. "Lipidomics" can be considered as a sub-field of metabolomics which aims to elucidate the biological processes in the context of lipids by measuring and characterizing the extended lipid profiles at the molecular level (lipidomic profiles). Traditional clinical lipid measures quantify total amounts of triglycerides, cholesterol, or lipoproteins. However, serum lipid profile is more complex at the molecular level. Current lipidomics platforms enable quantitative characterization of hundreds of diverse lipid molecular species across multiple lipid classes such as sphingolipids, phospholipids, sterol esters, acylglycerols, sterols, bile acids, fatty acids, eicosanoids, prostaglandins, and steroids [Seppaänen-Laakso, T and Ore 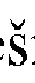 i 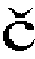 , M. 2009. How to study lipidomes. J Molec Endocr. 42: 185-190].

### SUMMARY OF THE INVENTION

In an aspect, the invention relates to the use of a prostaglandin selected from the group consisting of prostaglandin E2 (PGE2), prostaglandin F2 alpha (PGF2α), and combinations thereof, as a marker of endometrial receptivity for implanting an embryo.

In other aspect, the invention relates to a method for selecting the implantation window of an embryo in a human female based on the determination of the levels of said prostaglandins PGE2 and/or PGF2 in a sample of endometrial fluid from said human female.

In other aspect, the invention relates to method for assessing the fertility status of a human female.

In other aspect, the invention relates to a method for assessing if a human female subjected to *an in vitro* fertilization (IFV) process is under conditions suitable for receiving and implanting an embryo based on the determination of the level(s) of PGE2 and/or PGF2α in a sample of endometrial fluid from said human female, and correlating said level(s) of said prostaglandin(s) PGE2 and/or PGF2α with the conditions for receiving and implanting an embryo.

In other aspect, the invention relates to a kit comprising a reference lipid for prostaglandin E2 (PGE2) and/or for prostaglandin F2 alpha (PGF2α), and necessary reagents. The use of said kit for selecting the implantation window of an embryo in a human female, or for assessing the fertility status of a human female, or for assessing if a human female subjected to an IFV process is under conditions suitable for receiving and implanting an embryo, constitutes a further aspect of this invention.

In other aspect, the invention relates to a method for identifying a compound capable of enhancing endometrium receptivity.

In other aspect, the invention relates to a method for identifying a contraceptive agent.

In other aspect, the invention relates to a lipid composition, wherein said lipid composition is obtained from an endometrium fluid sample from a mammalian female during the implantation window of an embryo in said mammalian female. The use of said lipid composition for determining endometrial receptivity for implanting an embryo constitutes a further aspect of this invention.

In other aspect, the invention relates to a method for selecting the implantation window of an embryo in a mammalian female comprising the steps:
a) providing a sample of endometrial fluid from said mammalian female;
b) collecting a lipidomic profile from said sample; and
c) correlating said collected lipidomic profile with fertility status of a mammalian female.

### BRIEF DESCRIPTION OF DRAWINGS

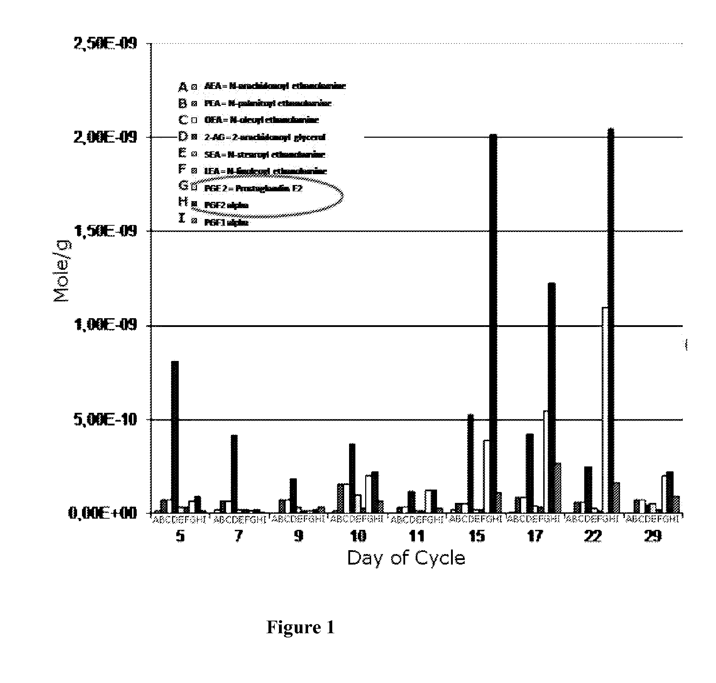

Figure 1 shows the levels of different lipids from endometrial fluid samples from women, during their natural menstrual cycle; the lipids were identified by liquid chromatography combined with tandem mass-spectrometry [A: N-arachidonoyl ethanolamine (AEA); B: N-palmitoyl ethanolamine (PEA); C: N-oleoyl ethanolamine (OEA); D: 2-arachidonoyl glycerol (2-AG); E: N-stearoyl ethanolamine (SEA); F: N-linoleoyl ethanolamine (LEA); G: prostaglandin E2 (PGE2); H: prostaglandin F2 alpha (PGF2α); I: prostaglandin F1 alpha (PGF1α)].

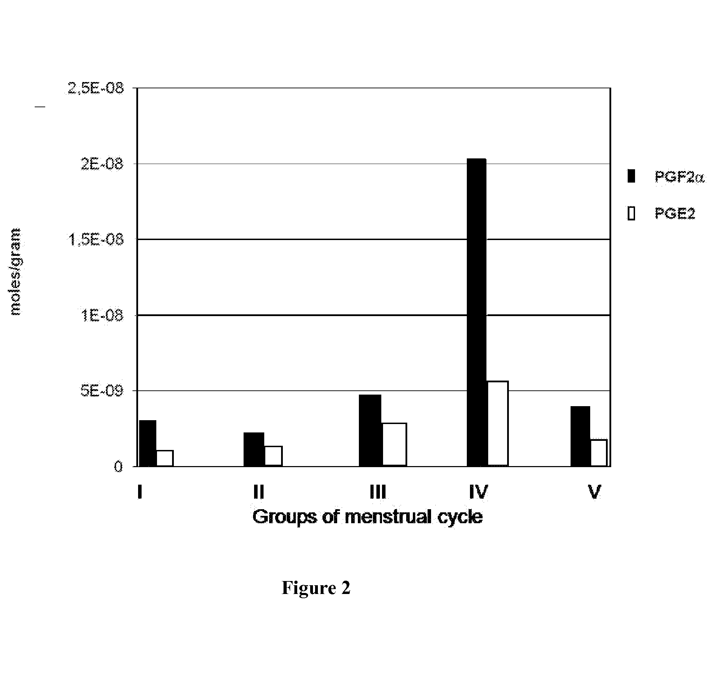

Figure 2 shows the levels of prostaglandin E2 (PGE2) and prostaglandin F2 alpha (PGF2α) in endometrial-fluid samples from women obtained throughout the menstrual cycle [Group I (days 0-8); Group II (days 9-14); Group III (days 15-18); Group IV (days 19-23) and Group V (days 24-30)].

### DETAILED DESCRIPTION OF THE INVENTION

An objective of the present invention is to provide a biomarker of endometrial receptivity for implanting an embryo.

The biomarker can be used for selecting the implantation window of an embryo in a mammalian female, preferably, in a human female (woman). Thus, said biomarker can also be used for assessing the fertility status of a mammalian female as well as for assessing if a mammalian female subjected to *in vitro* fertilization (IFV) is under conditions suitable for receiving and implanting an embryo. In a particular embodiment, said mammalian female is a human female (woman).

Further, the biomarker can be used to identify a compound capable of enhancing endometrium receptivity, or alternatively, to identify a contraceptive agent.

The inventors have now surprisingly found that lipid compounds can be used as biomarkers for endometrial receptivity for implanting an embryo. In particular, the inventors have analyzed the levels of some lipids in endometrial fluid samples between days 19 to 21 of the menstrual cycle of a human female (woman), coincident with the implantation window. Thus, it is now possible to identify the implantation window in a mammalian female by determining the lipid profile in endometrial fluid samples along the menstrual cycle. The method is based on comparison of the established lipid profile from a mammalian female to a reference lipidomic markers. In a preferred, particular embodiment, said mammalian female is a human female (woman).

In a particular embodiment, the inventors have observed that the concentration of two specific lipids, namely prostaglandin E2 (PGE2) and prostaglandin F2 alpha (PGF2α), is significantly increased in endometrial fluid samples between days 19 to 21 of the menstrual cycle of a human female (woman), coincident with the implantation window. Thus, it is now possible to identify the implantation window in a human female by determining the level of PGE2 and/or PGF2α in endometrial fluid samples along the menstrual cycle.

Thus, in an aspect, the invention relates to the use of a prostaglandin selected from the group consisting of prostaglandin E2 (PGE2), prostaglandin F2 alpha (PGF2α), and combinations thereof, as a marker of endometrial receptivity for implanting an embryo. In a particular embodiment, PGE2 and/or PGF2α can be used as a marker of endometrial receptivity for implanting an embryo in a mammalian female, preferably, in a human female.

PGE2, 7-[3-hydroxy-2-(3-hydroxyoct-1-enyl)-5-oxo-cyclopentyl]hept-5-enoic acid, is a naturally occurring prostaglandin, also known in medicine as "dinoprostone"; it has important effects in labour (softens cervix and causes uterine contraction) and also stimulates osteoblast to release factors which stimulate bone resoption by osteoclasts). Like other prostaglandins, dinoprostone can be used as an abortifacient since it is a direct vasodilator, relaxing smooth muscles, and it inhibits the release of noradrenaline from sympathetic nerve terminals.

PGF2α, (Z)-7-[(1R,2R,3R,5S)-3,5-dihydroxy-2-[(E,3S)-3-hydroxyoct-1-enyl) cyclopentyl]hept-5-enoic acid, is also a naturally occurring prostaglandin, also known in medicine as "dinoprost"; it is used in medicine to induce labour and as an abortifacient.

In another aspect, the invention relates to a method for selecting the implantation window of an embryo in a human female, hereinafter referred to as the "method of the invention", which comprises determining the level of PGE2, the level of PGF2α or the level of both PGE2 and PGF2α in a sample of endometrial fluid from said human female, wherein said implantation window is selected when the level of at least one of said prostaglandins PGE2 or PGF2α in said sample is increased in relation to a reference sample.

As used herein, the expression "method for selecting", relates to a method for determining the probability of a human female of being in a period receptive for embryo implantation. The skilled person in the art will observe that said prediction cannot be correct for the 100% of the patients under study. However, said expression requires that the prediction method provides correct results for a statistically significant portion of patients, what can be determined by using standard statistical techniques such as the confidence intervals determination, p value determination, t test of Student, or the Mann-Whitney test, as explained by Dowdy and Wearden, Statistics for Research, John Wiley & Sons, New York 1983. Suitable confidence intervals are, at least, 50%, at least 60%, at least 70%, at least 80%, preferably, at least 90%, or most preferable, at least 95%. Preferably, p values are 0.2, 0.1, or, most preferably 0.05.

As used herein, the term "implantation window" encompasses the window of time during which the uterine endometrium is receptive to the conceptus; in humans, this occurs in the secretory stage of the menstrual cycle. Implantation is defined as days 6-8 post the day of the luteinising hormone (LH) surge. The implantation window can be estimated on the basis of a regular menstrual pattern as approximately 7 days before the expected first day of the menstrual period, i.e., it corresponds, therefore, to days 19-21 of an ideal menstrual cycle of 28 days in humans. Similar cycles have been disclosed in other mammals, so that the method of the invention could be adapted to any mammalian female.

The levels of PGE2 and/or PGF2α are determined in a sample of endometrial fluid from the human female under study. The biological sample can be taken from said human female by conventional means. By illustrative, a sample of endometrial fluid can be obtained as mentioned in Example 1, i.e., by gently introducing transcervically an empty flexible catheter into the uterine cavity and gradually applying a suction with a syringe. Since one major challenge lies on the collection of samples of adequate purity, to prevent contamination by cervical mucus during catheter removal, the outer sheath of the embryo transfer catheter is advanced to an appropriate depth from the external cervical os, following the application of suction; then, cervical mucus is aspirated prior to the endometrial secretion aspiration. A satisfactory number of samples combined with good sample-collection practice will be sufficient to avoid false results.

The determination of the endometrium receptivity for embryo implantation is particularly important in a lot of techniques such as IVF, embryo transfer, etc., as well as for determining the optimum period for conception in couples who are trying to conceive in a natural way.

As used herein, the term "level" is intended broadly and can mean a quantitative amount (e.g., weight or moles), a semi-quantitative amount, a relative amount (e.g., weight % or mole % within class or a ratio), a concentration, and the like. In a particular embodiment, the levels of PGE2 and PGF2α are expressed in moles/gram of tissue [in that case, although samples were liquid, due to the small volume thereof, the samples were weighed and the concentrations of said compounds was expressed by reference to said weight - values were duly normalized].

The determination of the level of PGE2 and/or PGF2α in the biological sample can be determined by using any suitable method in view of the lipids to be detected and, optionally, quantified, for example, thin layer chromatography, gas chromatography, liquid chromatography (LC), mass spectrometry (MS) and NMR spectrometry, and any combination thereof (e.g., LC/MS or the like). In a particular embodiment, said lipids (PGE2 and PGF2α) were identified by liquid chromatography (LC) combined with tandem mass-spectrometry (MS) [LC/MS/MS]. Tandem mass-spectrometers include triple quadrupole, ion trap, and quadrupole/time-of-flight instruments, among others. These instruments typically use quadrupole technology to isolate a compound based upon its molecular weight prior to collision activation (fragmentation) and mass analysis of the fragmented components. This means that the mixture must be purified only to the point that the sample applied to the mass spectrometer is free of other compounds with the same mass. This can often be accomplished with a liquid-liquid extraction from the tissue followed by solid phase extraction methods. Quadrupole technology provides approximately 1 amu resolution; improved isolation within the mass spectrometer is accomplished using TOF/TOF instruments, which permits much finer resolution.

Alternative methods for determining the level of PGE2 and PGF2α from biological samples have been described, see, e.g., Voyksner & Bush, who disclosed that thermospray HPLC/MS analysis of the metabolites of arachidonic acid, including prostaglandins PGE2 and PGF2α among others, proved to be sensitive and specific [Voyksner, R.D. & Bush, E.D. Determination of prostaglandins, and other metabolites of arachidonic acid by thermospray HPLC/MS using post column derivatization. Biomedical and Environmental Mass Spectrometry, Volume 14, Issue 5:213-220 (published online: 13 Apr 2005)]; Surrenti et al., who disclosed a rapid and practical method for the separation and quantitation of PGE2 in human gastric juice by high performance liquid chromatography (HPLC) [Surrenti C. et al., High Performance Liquid Chromatographic Method for Prostaglandin E2 Determination in Human Gastric Juice Without Derivatization. Journal of Liquid Chromatography & Related Technologies, Volume 7, Issue 12 , October 1984, pages 2409 - 2419]; and Bastani et al., who developed an analytical method based on LC with negative electrospray ionization (ESI) coupled to tandem mass spectrometric detection (LC/MS/MS) for the determination of PGF2α derivatives concentrations in different biological samples [Bastani, N.E., et al. Determination of 8-epi PGF2α concentrations as a biomarker of oxidative stress using triple-stage liquid chromatography/tandem mass spectrometry. Rapid Communications in Mass Spectrometry, Volume 23 Issue 18, pages 2885 - 2890 (published Online: 10 Aug 2009)]. Another suitable method of detecting, and optionally quantifying, lipids in a biological sample employs stable isotope tracers to label the lipids.

In a particular embodiment, PGE2 and PGF2α levels are measured using liquid chromatography (LC) combined with tandem mass-spectrometry (MS) [LC/MS/MS].

The levels of PGE2 and/or PGF2α can be based on quantitative and/or semi-quantitative analysis. For example, semi-quantitative methods can be used to determine a level of a particular lipid (PGE2 or PGF2α) metabolite(s) above a threshold value or to determine ratios of different lipid metabolites, without assigning an absolute or relative numerical value. Quantitative methods can be used to determine a relative or absolute amount of a particular lipid metabolite(s) in the biological sample.

In semi-quantitative methods, a threshold or cutoff value can be determined by any means known in the art, and is optionally a predetermined value. In particular embodiments, the threshold value is predetermined in the sense that it is fixed, for example, based on previous experience with the assay and/or a population of human females. Alternatively, the term "predetermined" value can also indicate that the method of arriving at the threshold is predetermined or fixed even if the particular value varies among assays or may even be determined for every assay run.

The level of a prostaglandin such as PGE2 or PGF2α in the endometrial fluid sample from the human female under study is "increased in relation to" the level of said prostaglandins in a reference sample according to the instant invention, when the level of said prostaglandins in the endometrial fluid sample under study is at least, 1,1 -fold, 1,5-fold, 5-fold, 10-fold, 20-fold, 30-fold, 40-fold, 50-fold, 60-fold, 70-fold, 80-fold, 90-fold, 100-fold or even more with respect to the reference sample.

According to the method of the invention, the determination of the levels of PGE2 and/or PGF2α needs to be correlated to the level of said prostaglandins in a reference sample. As used herein, the term "reference sample", relates to an endometrium fluid sample obtained from a human female during the non-fertile period of said mammal. Due to the eventual variability which may exist among different human females as to the production of said prostaglandins during the non-fertile period, the reference sample can be typically obtained by combining the same amounts of samples of a population of human females. Generally, typical reference samples will be obtained from clinically well-documented human females. In said samples, normal concentrations (i.e., reference concentrations) of the biomarker (PGE2 and/or PGF2α) can be determined, by example, establishing the average concentration on the reference population. In order to determine the biomarker reference concentration, some considerations should be born in mind, e.g., age, etc. By illustrative, the same amounts of a group of at least 2, at least 10, at least 100, preferably, more than 1,000 human females, preferably sorted bearing in mind the above mentioned considerations (e.g., age, etc.) can be taken as the reference group.

In a particular embodiment, the reference sample is obtained from the endometrium fluid from a human female, or from a population of human females, during the non-fertile period of said human female(s). Although the reference sample can be obtained at any day during the non-fertile period of said human female(s), in a particular embodiment, said reference sample is obtained before the 15^{th} day of the menstrual cycle, typically between days 5-11 of the menstrual cycle. The predetermined value can be calculated by using the endometrial fluid samples of the menstrual cycle stage as defined above.

Alternatively, in order to obtain a more reliable determination of the implantation window, it is preferable to determine the levels of PGE2 and/or PGF2α along the menstrual cycle. Thus, the method of the invention can be performed daily, or each 2, 3, 4, 5, 6, 7, 8, 9, 10, 12, 13, 14, 15, 16, 17, 18, 19, 20, 21, 22, 23, 24, 25, 26, 27 or 28 days, so that the expression profile of said prostaglandins PGE2 and/or PGF2α can be determined along the full menstrual cycle. Typically, endometrium fluid samples can be obtained during the proliferative phase (days 1-14 of the menstrual cycle or before the LH peak), in the early secretory phase (days 15-19 of the menstrual cycle or days 1-5 after the LH peak), in the intermediate secretory phase (days 20-24 of the menstrual cycle or days 6-10 after the LH peak) and in the late secretory phase (days 25-28 of the menstrual cycle or days 11-14 after the LH peak). In this way, the optimal implantation window will correspond to the period of time in which the levels of PGE2 and/or PGF2α are the highest during the full menstrual cycle.

In a particular embodiment, the reference sample is obtained from the same human female under study during her non-fertile days in order to quantify the level(s) of said prostaglandins (PGE2 and/or PGF2α); said information can be used as a reference value.

In another particular embodiment, the reference sample is obtained from a generalised human females population during their non-fertile days and the level(s) of said prostaglandins (PGE2 and/or PGF2α) are quantified and combined to establish the reference value.

According to the present invention, bioactive lipids in endometrial secretions are analysed to determine their relative levels and establish a correlation with implantation and pregnancy outcome.

Thus, in other aspect, the invention relates to a method for assessing the fertility status of a human female wherein said method comprises:
- determining the level of PGE2, the level of PGF2α or the level of both PGE2 and PGF2α in a sample of endometrial fluid from said human female, and
- correlating the level of said prostaglandin(s) PGE2 and/or PGF2α with fertility status.

This method is aimed at assessing levels of said prostaglandin(s) PGE2 and/or PGF2α during the implantation window in the endometrium of said human female.

The PGE2 and/or PGF2α levels are correlated with endometrial receptivity and likelihood of conception.

In other aspect, the invention relates to a method for assessing if a human female subjected to an *in vitro* fertilization (IFV) process is under conditions suitable for receiving and implanting an embryo, wherein said method comprises:
- determining the level of PGE2, the level of PGF2α or the level of both PGE2 and PGF2α in a sample of endometrial fluid from said human female, and
- correlating the level of said prostaglandin(s) PGE2 and/or PGF2α with the conditions for receiving and implanting an embryo.

This method is also aimed at assessing levels of said prostaglandin(s) PGE2 and/or PGF2α during the implantation window in the endometrium of said human female.

The PGE2 and/or PGF2α levels are correlated with the conditions of the receiving human females for receiving and implanting an embryo and likelihood of conception.

The sample of endometrial fluid from said human female can be obtained at any time before implanting the embryo, e.g., from some minutes (e.g., 15 minutes) to some hours (e.g., 4-6 hours), or even some days (e.g., 1, 7, 14, or even more) before the embryo transfer. In a particular embodiment, this method is planned to be performed on the endometrial fluid obtained in a non-disruptive fashion 5-6 hours prior to embryo transfer.

As discussed above, the ability to identify specific lipid mediators in endometrial secretions that act as markers of endometrial receptivity is of great importance to the IVF process. By eliminating the necessity to obtain endometrial biopsies, a stressful step for the human female (patient) is avoided. Furthermore, correct endometrial dating is inaccurately obtained, despite the development of non-invasive techniques such as ultrasound ecography or magnetic resonance, which only provide with morphological information of the uterus that has proven not reliable. The present invention provides a method for determining the biochemical characteristics of the uterus and an appropriate receptive endometrium a few hours prior to embryo transfer, thus increasing the chances for successful embryo implantation. Thus, by optimizing the endometrial factor, the implantation rates could be improved by at least 5%, typically, at least 10%.

Another aspect of the invention is a kit comprising reference lipids for PGE2 and/or PGF2α (e.g., PGE2 and/or PGF2α lipids of known purity and concentration) and necessary reagents. The necessary reagents will be selected in function of the technique selected for determining the level(s) of PGE2 and/or PGF2α - the skilled person in the art knows the reagents which are necessary to perform a specific technique for determining the level of said lipids.

Said kit can be used for selecting the implantation window of an embryo in a human female, or for assessing the fertility status of a human female, or for assessing if a human female subjected to an IFV process is under conditions suitable for receiving and implanting an embryo. Thus, said kit can be used for an accurate analytical assessment of samples that are predictive of a receptive endometrium hours before the embryo transfer procedure.

Thus, as it has been previously mentioned, the invention provides a number of non-invasive diagnostic methods, and kits for performing said methods, based on the identification of the lipid profile in the endometrial fluid during the implantation window. In a particular embodiment, said lipid profile comprises PGE2 and/or PGF2α.

The methods provided by the instant invention are expected to increase current implantation rates by, at least 5%, preferably, at least 10%. An improvement of implantation rates is of benefit to both the medical community and patients by providing with a better medical assistance with increased chances of successful outcomes. By increasing pregnancy rates, a significant percentage of patients who must now go through a second round of treatment will avoid such a need, thus avoiding the anxiety and economic problem that it may represent.

Further, the methods and kits provided by the instant invention will provide the medical community with a powerful tool in the diagnosis of endometrial receptivity prior to embryo transfer in IVF treatments. Said methods will eliminate the necessity for invasive approaches, and will improve current implantation rates by offering real-time biochemical read-outs of endometrial receptivity only a few hours before the embryo transfer procedure. Patients consequently avoid upsetting biopsy-collection processes.

The invention further provides a method for identifying a compound capable of enhancing endometrium receptivity as well as a method for identifying a contraceptive agent.

Thus, in other aspect, the invention further provides a method for identifying a compound capable of enhancing endometrium receptivity, which comprises:
(i) contacting PGE2 and/or PGF2α producing cell with the compound under test (candidate compound), and
(ii) determining the level of said PGE2 and/or PGF2α,
wherein said candidate compound is suitable for enhancing endometrium receptivity if said compound causes an increase in the production of said PGE2 and/or PGF2α by said cell.

In other aspect, the invention further provides a method for identifying a contraceptive agent, which comprises:
(i) contacting a PGE2 and/or PGF2α producing cell with the compound under test (candidate compound), and
(ii) determining the level of said PGE2 and/or PGF2α,
wherein said candidate compound is suitable as a contraceptive agent if said compound causes a decrease in the production of said PGE2 and/or PGF2α by said cell.

In both cases, the candidate compound to be tested in said method for identifying a compound capable of enhancing endometrium receptivity or for identifying a contraceptive agent, can be practically any chemical compound, including both low molecular weight compounds and macromolecules such as proteins, nucleic acids, lipids and the like. The expression "contacting", as used herein, includes any possible way of introducing the candidate compound into said PGE2 and/or PGF2α producing cells.

Although virtually any PGE2 and/or PGF2α producing cell can be used in the working of said methods (for identifying a compound capable of enhancing endometrium receptivity or for identifying a contraceptive agent), in a particular embodiment, said PGE2 and/or PGF2α producing cell can be an epithelial cell of the endometrium [epithelial endometrium cell], preferably, a human epithelial endometrium cell; in another particular embodiment, said PGE2 and/or PGF2α producing cell is cell line from endometrium, preferably, a human cell line from endometrium, said cell having the ability of producing PGE2 and/or PGF2α. In this case, the level of said PGE2 and/or PGF2α can be determined in the culture medium by any of the techniques previously mentioned. Alternatively, it is also possible that said PGE2 and/or PGF2α producing cells are present in an animal model; then, the level of said PGE2 and/or PGF2α can be determined in a sample from the endometrium fluid from said animal model by any of the techniques previously mentioned.

An increase in the production of said PGE2 and/or PGF2α may be indicative that the candidate compound is suitable for potentially enhancing or increasing the endometrium receptivity for implanting an embryo.

Similarly, a decrease in the production of said PGE2 and/or PGF2α may be indicative that the candidate compound can be used as a contraceptive agent since said compound reduces the endometrium receptivity for implanting an embryo.

In a further aspect, the invention relates to a lipid composition, wherein said lipid composition has been obtained from an endometrium fluid sample from a mammalian female during the implantation window of an embryo in said mammalian female. Said lipid composition can be used for determining endometrial receptivity for implanting an embryo.

The term "mammalian", as used herein, relates to any mammal, which includes but is not limited to humans, non-human primates, cattle, goats, sheeps, horses, pigs, dogs, etc., subjects. Preferably, the mammalian is a human being, and, thus, the mammalian female is a woman. In a particular embodiment, said lipid composition is obtained from an endometrium fluid sample from a human female during the implantation window of an embryo in said human female.

Further, in a particular embodiment, said lipid composition comprises a lipid selected from the following lipids N-arachidonoyl ethanolamine (AEA), N-palmitoyl ethanolamine (PEA), N-oleoyl ethanolamine (OEA), 2-arachidonoyl glycerol 2-AG), N-stearoyl ethanolamine (SEA), N-linoleoyl ethanolamine (LEA), prostaglandin E2 (PGE2), prostaglandin F2 alpha (PGF2α), prostaglandin F1 alpha (PGF1α) and combinations thereof. In a preferred embodiment, said lipid composition comprises a prostaglandin selected from the group consisting of PGE2, PGF2α, and combinations thereof because the levels of said specific lipid(s) is/are significant increased in endometrial fluid samples between days 19 to 21 of the menstrual cycle of a human female (woman), coincident with the implantation window.

Thus, in another aspect, the invention relates to a method for selecting the implantation window of an embryo in a mammalian female comprising the steps:
d) providing a sample of endometrial fluid from said mammalian female;
e) collecting a lipidomic profile from said sample; and
f) correlating said collected lipidomic profile with fertility status of a mammalian female.

The sample of endometrial fluid from the mammalian female under study can be obtained by conventional methods as discussed above.

Obtaining a lipidomic profile from said sample of endometrium fluid from the mammalian female under study can be performed with various chemical and high resolution analytical techniques. Suitable analytical techniques include but are not limited to mass spectrometry (MS) and nuclear resonance spectroscopy (NRS). Any high resolution technique capable of re-solving individual lipids or lipid classes and provides structural information of the same can be used to collect the lipid profile from said biological sample. Collecting the lipidomic profile with mass spectrometry (MS) is one embodiment of the current invention. The MS instrument can be coupled to a high performance separation method such as HPLC.

The analytical technique used for collecting the lipidomic profile should be able to quantify or measure either the exact amount or at least a relative amount of the individual lipids or lipid classes. The amount of the individual lipids or lipid classes in the collected lipidomic profile is used when comparing the collected lipid profile to the reference lipidomic biomarkers. In a particular embodiment, the levels of the lipids are determined by LC/MS/MS, as discussed below.

The reference lipidomic biomarkers can be established from the same mammalian female under study or it can be from a generalised population. If the same mammalian females are used to create the reference lipidomic marker, then a sample could be collected from said mammalian female during the non-fertile days thereof. The reference lipidomic marker is then created from that first lipid profile of that mammalian female. This lipidomic marker is used as a base-line or starting point. A series of lipidomic profiles can be collected along the menstrual cycle. These lipidomic profiles are then compared with the reference lipidomic marker previously created.

The reference lipidomic markers can also be created from a generalized population of mammalian females. If a generalized population is used then several lipid profiles from said are combined and the lipidomic marker is created from this combination.

In a particular embodiment, the reference lipidomic markers are one or more lipid(s) selected from the following lipids N-arachidonoyl ethanolamine (AEA), N-palmitoyl ethanolamine (PEA), N-oleoyl ethanolamine (OEA), 2-arachidonoyl glycerol (2-AG), N-stearoyl ethanolamine (SEA), N-linoleoyl ethanolamine (LEA), prostaglandin E2 (PGE2), prostaglandin F2 alpha (PGF2α), prostaglandin F1 alpha (PGF1α) and combinations thereof. In a preferred embodiment, said lipid profile comprises a prostaglandin selected from the group consisting of PGE2, PGF2α, and combinations thereof because the levels of said specific lipid(s) is/are significant increased in endometrial fluid samples between days 19 to 21 of the menstrual cycle of a human female (woman), coincident with the implantation window.

Another aspect of the invention is a kit comprising reference lipids to form the lipidomic reference biomarkers and necessary reagents.

Said kit can be used for selecting the implantation window of an embryo in a mammalian female, or for assessing the fertility status of a mammalian female, or for assessing if a mammalian female subjected to IFV is under conditions suitable for receiving and implanting an embryo. Thus, said kit can be used for an accurate analytical assessment of samples that are predictive of a receptive endometrium hours before the embryo transfer procedure.

The following examples illustrate the invention but are not intended to limit the scope of the invention.

### EXAMPLE 1

### The concentration of PGE2 and PGF2α is significantly increased during the window of implantation

### 1.1 Materials

HPLC-grade methanol and acetonitrile used for mass spectrometric studies were purchased from VWR international (Plainview, NY). HPLC grade water, mass spectrometry/HPLC grade acetic acid, formic acid, and ammonium acetate were purchased from Sigma-Aldrich (St. Louis, MO).

### 1.2 Methodology

**Design.** Inventors conducted a single-blind study in 39 healthy female donors after obtaining informed, signed consent. All samples were collected from women during their natural menstrual cycle; endometrial aspirates were assigned an identification number and preserved using standard methods prior analysis.

**Endometrial secretion aspiration procedure.** With the patient lying in lithotomy position, the cervix was cleansed after insertion of the speculum. An empty flexible catheter (Wallace, Smith Medical International) was gently introduced 6 cm transcervically into the uterine cavity and suction was gradually applied with a 10 ml syringe. To prevent contamination by cervical mucus during catheter removal, the outer sheath of the embryo transfer catheter was advanced to a depth of 4 cm from the external cervical os, following the application of suction. Cervical mucus was aspirated prior to endometrial secretion aspiration for within patient comparison, in order to verify whether the aspirates represented cervical mucus rather than endometrial secretions.

**Sample analysis.** Lipids from endometrial-fluid extracts were identified by liquid chromatography (LC) combined with tandem mass-spectrometry (MS). HPLC grade water was added to the samples to make a 30% organic solution. Lipids were partially purified on C18 solid phase extraction columns as previously described [Bradshaw, HB., Rimmerman, N., Krey, JF and Walker, JM. 2006. Sex and hormonal cycle differences in rat brain levels of pain-related cannabimimetic lipid mediators. Am J Physiol Regul Integr Comp Physiol. 291: R349-358]. In brief, each 500 mg column was conditioned with 5 ml methanol and 2.5 ml water followed by loading of the water/supernatant solution. Columns were then washed with 2 ml water and 1.5 ml 55% methanol. Compounds were eluted with 1.5 ml methanol. Eluants were vortexed at maximum speed prior to mass spectrometric analysis.

Tandem mass-spectrometers include triple quadrupole, ion trap, and quadrupole/time-of-flight instruments, among others. These instruments typically use quadrupole technology to isolate a compound based upon its molecular weight prior to collision activation (fragmentation) and mass analysis of the fragmented components. This means that the mixture must be purified only to the point that the sample applied to the mass spectrometer is free of other compounds with the same mass. This can often be accomplished with a liquid-liquid extraction from the tissue followed by solid phase extraction methods. Quadrupole technology provides approximately 1 amu resolution; improved isolation within the mass spectrometer is accomplished using TOF/TOF instruments, which permit much finer resolution.

Specifically, rapid separation of analytes was obtained using 10 µl injections (Agilent 1100 series autosampler, Wilmington, DE) onto a Zorbax eclipse XDB 2.1 × 50 mm reversed phase column. Gradient elution (200 µl/min) was formed under pressure on a pair of Shimadzu (Columbia, Maryland) 10AdVP pumps. Mass spectrometric analysis was performed with an Applied Biosystems/MDS Sciex (Foster City, CA) API 3000 triple quadrupole mass spectrometer equipped with an electrospray ionization source. Levels of each compound were analyzed by multiple reactions monitoring (MRM) on the LC/MS/MS system.

**Mass spectrometric quantitation.** The quantitation of analytes was achieved using Analyst software (Applied Biosystems-MDS Sciex; Framingham MA), which quantifies the amount of analyte in the sample based upon a power fit of a linear regression of known concentrations of synthetic standards. Statistical differences were determined using ANOVA with post-hoc Fisher's LSD using a 95% confidence interval for the mean (SPSS software, Chicago, IL).

### 1.3 Results

A total of 39 endometrial fluid samples obtained throughout the menstrual cycle [Group I (days 0-8) (n=8); Group II (days 9-14) (n=8); group III (days 15-18) (n=8); Group IV (days 19-23) (n=8) and group V (days 24-30) (n=7)] were analysed for changes in lipid concentration in two single-blinded independent experiments.

In the first experiment (n=13), inventors found a significant increase in the concentration of two specific lipids (PGE2 and PGF2α) between days 19 to 21 of the menstrual cycle, coincident with the window of implantation. None of the remaining lipids identified in the samples [N-arachidonoyl ethanolamine, N-palmitoyl ethanolamine, N-oleoyl ethanolamine, 2-arachidonoyl glycerol, N-stearoyl ethanolamine, N-linoleoyl ethanolamine, PGF1α] underwent significant changes during the menstrual cycle (Figure 1).

A second experiment (n=26) confirmed the peak of the same lipids reported in the first experiment. The combined results from both experiments (n=39) demonstrate a 2-fold and 20-fold increase peak in the concentration of each lipid, respectively, during the clinical window of implantation (Figure 2).

These results suggest that PGE2 and/or PGF2α could be important markers of human endometrial receptivity during the window of implantation.

## Claims

1. Use of a prostaglandin selected from the group consisting of prostaglandin E2 (PGE2), prostaglandin F2 alpha (PGF2α), and combinations thereof, as a marker of endometrial receptivity for implanting an embryo.

2. A method for selecting the implantation window of an embryo in a human female, which comprises determining the level of PGE2, the level of PGF2α or the level of both PGE2 and PGF2α in a sample of endometrial fluid from said human female, wherein said implantation window is selected when the level of at least one of said prostaglandins PGE2 or PGF2α in said sample is increased in relation to a reference sample.

3. A method for assessing the fertility status of a human female wherein said method comprises:
- determining the level of PGE2, the level of PGF2α or the level of both PGE2 and PGF2α in a sample of endometrial fluid from said human female, and
- correlating the level of said prostaglandin(s) PGE2 and/or PGF2α with fertility status.

4. A method for assessing if a human female subjected to *an in vitro* fertilization (IFV) process is under conditions suitable for receiving and implanting an embryo, wherein said method comprises:
- determining the level of PGE2, the level of PGF2α or the level of both PGE2 and PGF2α in a sample of endometrial fluid from said human female, and
- correlating the level of said prostaglandin(s) PGE2 and/or PGF2α with the conditions for receiving and implanting an embryo.

5. A kit comprising a reference lipid for prostaglandin E2 (PGE2) and/or for prostaglandin F2 alpha (PGF2α), and necessary reagents.

6. The use of a kit according to claim 5, for selecting the implantation window of an embryo in a human female, or for assessing the fertility status of a human female, or for assessing if a human female subjected to *an in vitro* fertilization (IFV) process is under conditions suitable for receiving and implanting an embryo.

7. A method for identifying a compound capable of enhancing endometrium receptivity, which comprises:
(i) contacting PGE2 and/or PGF2α producing cell with the compound under test (candidate compound), and
(ii) determining the level of said PGE2 and/or PGF2α,
wherein said candidate compound is suitable for enhancing endometrium receptivity if said compound causes an increase in the production of said PGE2 and/or PGF2α by said cell.

8. A method for identifying a contraceptive agent, which comprises:
(i) contacting a PGE2 and/or PGF2α producing cell with the compound under test (candidate compound), and
(ii) determining the level of said PGE2 and/or PGF2α,
wherein said candidate compound is suitable as a contraceptive agent if said compound causes a decrease in the production of said PGE2 and/or PGF2α by said cell.

9. A lipid composition, wherein said lipid composition is obtained from an endometrium fluid sample from a mammalian female during the implantation window of an embryo in said mammalian female.

10. Use of a lipid composition according to claim 9, for determining endometrial receptivity for implanting an embryo.

11. A method for selecting the implantation window of an embryo in a mammalian female comprising the steps:
a) providing a sample of endometrial fluid from said mammalian female;
b) collecting a lipidomic profile from said sample; and
c) correlating said collected lipidomic profile with fertility status of a mammalian female.
